**Europäisches Patentamt**

**European Patent Office**

**Office européen des brevets**

(19)

(11) Veröffentlichungsnummer: **0 002 169**
**B1**

(12) # EUROPÄISCHE PATENTSCHRIFT

(45) Veröffentlichungstag der Patentschrift: **09.09.81**

(21) Anmeldenummer: **78810021.2**

(22) Anmeldetag: **10.11.78**

(51) Int. Cl.³: **A 61 F 5/42**

(54) Erektionshalter.

(30) Priorität: **11.11.77 CH 13764/77**

(43) Veröffentlichungstag der Anmeldung:
**30.05.79 Patentblatt 79/11**

(45) Bekanntmachung des Hinweises auf die Patenterteilung:
**09.09.81 Patentblatt 81/36**

(84) Benannte Vertragsstaaten:
**BE DE FR GB NL SE**

(56) Entgegenhaltungen:
**DE - C - 554 178**
**US - A - 3 455 301**

(73) Patentinhaber: **Gasser, François W.**
**Mühlemattstrasse 57**
**CH-3007 Bern (CH)**

(72) Erfinder: **Hanus, Judith**
**Röhrliberg 36**
**CH-6330 Cham (CH)**

Courier Press, Leamington Spa, England.

## Erektionshalter

Die vorliegende Erfindung betrift einen Erektionshalter als Hilfsmittel für die Ermöglichung und/oder Verlängerung der Erektion des männlichen Gliedes, insbesondere bei impotenten oder potenzschwachen Männern.

Der Geschlechtsakt, bei dem beide Partner zum Orgasmus und zu völliger Befriedigung gelangen, ist für das psychische und physische Gleichgewicht und Wohlbefinden des Menschen von grösster Wichtigkeit. Insbesondere für den Mann ist es für die Stärkung und Erhaltung seines Selbstvertrauens wesentlich, dass er die Sicherheit hat, seine Frau jederzeit befriedigen und glücklich machen zu können. Die heutige Lebensweise mit ihren häufigen Stressituationen fordert aber gerade in dieser Richtung immer mehr Opfer, indem überbeanspruchte oder überforderte Männer häufig nicht mehr in der Lage sind, sich genügend zu entspannen, um einen befriedigenden Geschlechtsakt zu vollziehen. Impotenz oder Potenzschwäche sind die direkten Folgen, mit all ihren negativen Auswirkungen auf das Verhältnis zwischen Mann und Frau.

Dieser Problemkreis ist seit langem bekannt. Es ist auch bereits mehrmals versucht worden, Hilfsmittel zu entwickeln und in Verkehr zu bringen, mittels welchen die ungenügende oder sogar ganz fehlende Erektionsfähigkeit des männlichen Gliedes behoben, respektive überdeckt werden kann. Die diversesten Teil- oder Ganzprothesen, die aber alle entweder unangenehm im Gebrauch oder unästhetisch sind, sind heute bekannt. Als beispiel sei hier nur die Vorrichtung nach der Deutschen Offenlegungsschrift Nr. 24 60 812 vom 1. Juli 1976 von Leonhard Schmid erwähnt.

Ausserdem sind Erektionshalter bekannt, die aus einer elastischen, etwa 5 cm langen Manschette bestehen. Die Form der Manschette ist im allgemeinen zylindrisch und die Basis, die das Glied an seiner Wurzel abschliesst, ist steifer als das äussere Ende, wobei die höhere Steifheit entweder durch grössere Dicke der Wand oder durch Anwendung eines Materials mit einem höheren Elastizitätsmodul verursacht wird. Die Manschette kann ausserdem radiale Erhebungen besitzen. Solch ein Erektionshalter ist beispielsweise in der amerikanischen Patentschrift Nr. 3,455,301 (Clark) beschrieben.

Der Zweck von solchen Einrichtungen ist, Blutstauung hervorzurufen, wobei die elastische Manschette als ein Blutstauventil wirkt. Der Durchfluss darf jedoch aus naheliegenden Gesundheitsgründen nicht ganz eingestellt werden und es ist ausserordentlich schwierig das erwünschte Optimum der Wirkung mindestens annähernd zu schaffen. Aus diesem Grunde wird in der obenerwähnten amerikanischen Patentschrift betont, dass die zylindrische Manschette an ihrem äusseren Ende am leichtesten deformierbar sein soll, so dass sie durch achsiale Drücke beim Gebrauch erweitert wird. Dabei wird die Lichtweite der Manschette periodisch gegen die Basis verjüngt und durch die derart erhöhte vorübergehende Stauwirkung soll die unerwünschte Wirkung der gesagten Drücke, die sonst die Erektion schwächte, kompensiert werden.

Es hat sich herausgestellt, dass bei bisherigen Einrichtungen eine ganz zuverlässige Wirkung nur dann erreicht wird, wenn sämtliche Faktoren, insbesondere die erforderliche Deformationskraft an einzelnen Stellen sowie der Durchmesser der Manschette den physiologischen Faktoren und Bedingungen des Trägers tunlichst genau angepasst werden. Die daraus folgende hohe Anzahl von Typen kompliziert und verteuert die Erzeugung beträchtlich und macht gleichzeitig die Wahl des passenden Modells schwierig. Es wäre daher wünschenswert, den Erektionshalter derart zu vervollkommnen, dass die Grenzen der oben erwähnten Faktoren, innerhalb von welchen eine zuverlässige Wirkung erreicht wird, erweitert werden. Dadurch wird die notwendige Anzahl von Typen bedeutend beschränkt und der Herstellungspreis herabgesetzt.

Der vorliegenden Erfindung liegt die Aufgabe zugrunde, einen Erektionshalter der eingangs erwähnten Art zu schaffen, der keinen der Nachteile der bekannten Vorrichtungen aufweist, d.h. eine raschere und zuverlässigere Erektion gewährleistet, und der einfach, ästhetisch und sicher im Gebrauch ist, und der das männliche Glied in kurzer Zeit zu voller Erektion bringt und es, auch nach erfolgter Ejakulation längere Zeit voll erigiert hält und gleichzeitig automatisch mithilft, die Clitoris der Frau in natürlicher Art und Weise zu reizen. Im weiteren soll der erfindungsgemässe Erektionshalter sowohl für den Mann als auch für die Frau im Gebrauch angenehm und nicht störend sein. Er soll zudem beliebig oft verwendbar, völlig geruchsneutral und gegen die Ausscheidungen im Genitalbereich von Mann und Frau unempfindlich sein.

Erfindungsgemäss wird die gestellte Aufgabe dadurch gelöst, dass nur zwei radiale Erhebungen vorgesehen sind, von denen die eine an dem einen Manschettenende vorgesehen und die andere Erhebung dem anderen Manschettenende benachbart ist, und dass die Erhebung, die das Glied an seiner Wurzel umschliesst, mit einer zum Körper des Trägers hin gerichteten Verlängerung in Form einer radial wegstehenden Lippe versehen ist, wobei der zwischen den beiden Erhebungen liegende, eine Stauzone bildende Teil der Manschette nach aussen gewölbt ist.

Die besagte Erhebung weist vorzugsweise eine innere stumpfe Kante auf, die im achsialen Schnitt gesehen in die kon-kav ausgebildete Innenseite der radialen Lippe übergeht. Der Krümmungshalbmesser der Kante der radialen Erhebung beträgt vorzugsweise 3—6 mm.

Die Wirkungsweise der erfindungsgemässen Konstruktion besteht darin, dass beide Erhebungen auf das erigierte Glied als Blutstauventile mit unterschiedlicher Staukraft und die Lippe an der Gliedwurzel als Saugpumpe wirken.

Vorteilhafterweise ist der erfindungsgemässe Erektionshalter achsialsymmetrisch und weist aussen eine fassähnliche Form auf, die an beiden Enden geöffnet ist. Er besteht aus einem gesundheitsunschädlichen, geruchsfreien, weichen Elastomer, das gegebenenfalls antibakteriell wirkende Substanzen enthalten kann und vorzugsweise hautfarben gefärbt ist. Als Beispiele von geeigneten Elastomeren dienen verschiedene Arten von Silikongummi, weicher natürlicher oder synthetischer Gummi, der keine aktiven Füllmittel und keine schleimhautreizenden Zusatzstoffe wie Vulkanisationsbeschleuniger enthält sowie ferner mit gesundheitsundschädlichen, nicht reizenden Weichmachern plastifizierte Polymere und Kopolymere von Vinylchlorid, Vinylidenchlorid, Akrylnitril und ähnliches, Butylkautschuk, Polyesteramide, Polyurethane. Diese Aufstellung ist natürlich nicht ausschöpfend und die Erfindung ist auf die Anwendung der genannten Elastomere nicht beschränkt.

Weitere Einzelheiten des erfindungsgemässen Erektionshalters sowie seine Verwendung und Wirkungsweise gehen aus der nachfolgenden Beschreibung einer Ausführungsform hervor. Die Beschreibung stützt sich dabei auf die Zeichnung, die in

Fig. 1 eine perspektivische Ansicht dieser Ausfürungsform, in

Fig. 2 einen Längsschnitt durch diese Ausfürungsform und in

Fig. 3 einen auf einem erigierten Glied sitzenden erfindungsgemässen Erektionshalter im Schnittzeigt.

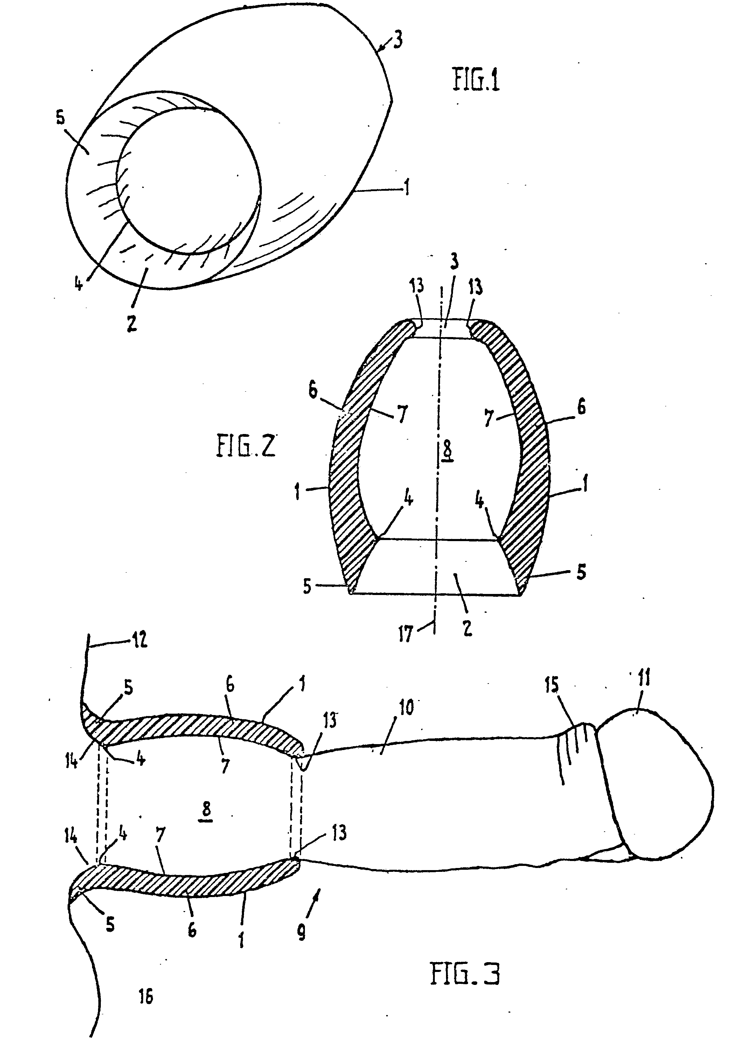

Man ersieht aus Fig. 1 die ungefähr tonnenförmige Gestaltung der Aussenseite 1 des Erektionshalters, dessen untere Oeffnung 2 einen leicht grösseren Durchmesser aufweist als die obere Oeffnung 3. Ferner erkennt man eine innere Erhebung 4, an die sich achsial die radiale Lippe 5 anschliesst.

Fig. 2 zeigt die Formgebung der Wandung 6 des Erektionshalters, dessen Aussenseite 1 im mittleren Bereiche des Erektionshalters, nahezu parallel zu der Innenseite 7 verläuft. Besagter Bereich wirkt als Stauraum 8, in dem überschüssiges Blut im Phallus 9 (Fig. 3) das in dessen Schwellkörper im vorderen Teil 10, an welchen die Eichel 11 anschliesst, nicht mehr Platz hat, aber durch die Lippe 5 aus den Arterien im Unterleib 12 in das Glied gepumpt worden ist, gestaut wird. Um die obere Oeffnung 3 herum läuft eine vordere Erhebung 13, die den Stauraum 8 gegen vorne hin begrenzt, ähnlich wie dies die innere Erhebung 4 gegen hinten tut.

Anhand von Fig. 3 lässt sich die Funktion des erfindungsgemässen Erektionshalters erläutern. Vorteilhaftweise wird er, nachdem seine Innenseite 7 mit einer fettfreien Salbe eingerieben worden ist, über das schlaffe Glied gestülpt und nach Möglichkeit in einem Zug bis ganz an die Wurzel 14 des Gliedes gezogen. Dabei ist darauf zu achten, dass die Haut des Gliedes ganz nach vorne aus dem Erektionshalter herausgezogen wird, um diesem die Möglichkeit zu nehmen, auf der Haut auf dem prallen Phallus 9 hin und her zu rutschen. Soweit vorhanden muss die Vorhaut 15 bei erigiertem Glied ihre natürliche Lage einnehmen. Die radiale Lippe 5 soll ganz am Unterleib 12 und am Hodensack 16 anliegen, so dass sich die innere Erhebung 4 in unmittelbarer Nähe der Gliedwurzel 14 befindet.

Sofern der erfindungsgemässe Erektionshalter derart auf das Glied aufgesetzt ist, bewirkt jede seiner Bewegungen, die durch die Bewegungen des Phallus 9 bewirkt werden, dass die Lippe 5 ihre ursprüngliche Stellung einzunehmen versucht und sich gegen die Symmetrieachse 17 (Fig. 2) hin zu schliessen versucht. Dadurch wird sich in den Adern im Unterleib 12 und der Gliedwurzel 14 befindliches Blut in den unteren Teil des Gliedes und somit in den Stauraum 8 gedrückt. Die innere Erhebung 4 verhindert dann, dass es widerstandslos wieder aus den Schwellkörpern im Phallus 9 abfliessen kann. Dadurch erigiert sich das Glied sehr schnell, und sein vorderer Teil 10 wird steif. Sobald dies geschehen ist, verhindert die vordere Erhebung 13, die den Erektionshalter gegen vorne abschliesst, dass das sich in den Schwellkörpern des vorderen Teiles 10 des Phallus 9 befindliche Blut wieder nach hinten wegfliesst. Das Glied bleibt somit erigiert. Dies selbst nach einer eventuellen Ejakulation, nach welcher sich üblicherweise die Schwellkörper sehr rasch entleeren und der Mann nicht mehr in der Lage ist, den Geschlechstakt weiterzuführen, selbst wenn seine Partnerin noch nicht zum Orgasmus gekommen ist.

Bei korrekter Dimensionierung und Anpassung des erfindungsgemässen Erecktionshalters an die anatomischen Gegebenheiten des männlichen Gliedes, verhilft er selbst einem zu einer normalen Erektion nicht mehr fähigen, impotenten Mann zu einem prall gefüllten Phallus, der in der Regel länger und dicker sein wird, als bei einer normalen Erektion. Die Erfindung ermöglicht es auch jedem Manne, nach Wunsch sein Glied mehrere Stunden voll erigiert zu erhalten. Ein voll befriedigendes Sexualverhalten ist somit dank dem erfindungsgemässen Erektionshalter jedem Manne wieder möglich.

Im weiteren erleichtert der erfindungsgemässe Erektionshalter selbst der Frau das Erreichen des Orgasmus, reibt doch die Aussenseite 1 des Erektionshalters als verdicktes Teil des Phallus in natürlicher Art und Weise die weibliche Clitoris.

Das Abstreifen des erfindungsgemässen Erektionshalters kann in einfacher Weise dadurch geschehen, dass, wenn vorhanden, die Vorhaut 15 über die Eichel 11 gezogen und das erigierte Glied in den Erektionshalter hineingedrdrückt wird, worauf es sehr rasch erschlafft, so das letzterer problemlos abgezogen werden kann.

Der erfindungsgemässe Erektionshalter kann, zur weiteren Unterstützung seiner positiven Wirkung mit zusätzlichen Hilfsmitteln, wie beispielsweise einem Hodenband oder einem Reizelement für die Frau, verbunden werden.

**Patentansprüche**

1. Erektionshalter als Hilfsmittel für die Ermöglichung und/oder Verlängerung der Erektion des männlichen Gliedes in der Form einer Manschette, die aus beschränkt dehnungsfähigem, elastischem Material besteht und nach innen vorstehende radiale Erhebungen aufweist, wobei die Basis der Manschette einen höheren Deformationswiderstand als das äussere Ende besitzt, dadurch gekennzeichnet, dass nur zwei radiale Erhebungen (4, 13) vorgesehen sind, von denen die eine an dem einen Manschettenende vorgesehen und die andere Erhebung dem anderen Manschettenende benachbart ist, und dass die Erhebung (4), die das Glied an seiner Wurzel umschliesst, mit einer zum Körper des Trägers hin gerichteten Verlängerung in Form einer radial wegstehenden Lippe (5) versehen ist, wobei der zwischen den beiden Erhebungen liegende, eine Stauzone (8) bildende Teil der Manschette nach aussen gewölbt ist.

2. Erektionshalter nach Anspruch 1, dadurch gekennzeichnet, dass die das Glied an seiner Wurzel umschliessende radiale Erhebung (4) einen grösseren Innendurchmesser als die radiale Erhebung (13) besitzt.

3. Erektionshalter nach den Ansprüchen 1 und 2, dadurch gekennzeichnet, dass das Minimum des Deformationswiderstandes der Manschettenwand in der Stauzone (8) liegt.

4. Erektionshalter nach Anspruch 1, dadurch gekennzeichnet, dass die radiale Erhebung (4) eine innere stumpfe Kante aufweist, die im achsialen Schnitt gesehen in die konkav ausgebildete Innenseite der radialen Lippe (5) stufenlos übergeht.

5. Erektionshalter nach Anspruch 4, dadurch gekennzeichnet, dass der Krümmungshalbmesser der Kante der radialen Erhebung (4) 3—6 mm beträgt.

**Claims**

1. Erection holder destined as an auxiliary means for enabling and/or prolonging an erection of the penis of a man, which holder is of a sleeve-like configuration and consists of an elastic material with limited extensibility, said holder comprising radial protrusions directed inwardly, whereby the base end of said configuration presents a higher resistance to deformation than the opposite outer end of it, characterized in that only two radial protrusions (4, 13) are provided, one of which is located at the end of the configuration whereagainst the other is located near the opposite end of the configuration, whereby the protrusions (4) surrounding the root of the penis is provided with an extension directed towards the abdomen of the user and presenting the form of a radially extending lip (3), the whole in such a way, that the part of the configuration located between the two protrusions which form a reservoir zone (8) is curved outwardly.

2. Erection holder according to claim 1, characterized in that the radial protrusion (4) surrounding the root of the penis presents a larger inner diameter than does the other radial protrusion (13).

3. Erection holder according to claims 1 and 2, characterized in that the minimum of deformation resistance of the wall of the configuration is located in the reservoir zone (8).

4. Erection holder according to claim 1, characterized in that the radial protrusion (4) has a blunt inwardly facing edge which, in the axial section, presents a concave inner side merging into the radial lip (5).

5. Erection holder according to claim 4, characterized in that the radius of the curvature of said edge of the radial protrusion (4) is of about 3 to 6 mm.

**Revendications**

1. Support d'érection comme moyen auxiliaire pour permettre et/ou prolonger l'érection du membre masculin en forme d'une manchette faite de matière élastique d'un extensibilité limitée, présentant des élévations radiales intérieures, la base de la manchette présentant une résistance à la déformation plus élevée que sa partie extérieure, caractérisé en ce que uniquement deux élévations (4, 13) sont prévues, dont l'une se trouve à un bout de la manchette et l'autre près de l'autre bout de la manchette, et en ce que l'élévation (4) entourant la partie arrière du membre est équipée d'un prolongement dirigé en direction du corps de l'utilisateur en forme d'une lèvre radiale (5), le tout de sorte que la partie de la manchette se trouvant entre les deux élévations formant une zone de réservoir est bombée contre l'extérieur.

2. Support d'érection selon la revendication 1, caractérisé en ce que l'élévation radiale (4) entourant la partie arrière du membre présente un diamètre intérieur supérieur à celui de l'élévation radiale opposée (13).

3. Support d'érection selon les revendications 1 et 2, caractérisé en ce que le minimum de résistance à la déformation de la paroi de la manchette se situe dans la zone de réservoir (8).

4. Support d'érection selon la revendication 1, caractérisé en ce que l'élévation (4) présente une arrête intérieure émoussée mergeant, dans la coupe axiale, dans la partie intérieure concanve de la lèvre radiale (5).

5. Support d'érection selon la revendication 4, caractérisé en ce que le rayon de courbure de l'arrête de l'élévation radiale (4) est de 3 à 6 mm.

0 002 169

FIG.1

FIG.2

FIG.3